# EUROPEAN PATENT APPLICATION

(11) **EP 2 860 688 A1**
(43) Date of publication of application: **15.04.2015**
(21) Application number: 13803892.2
(22) Date of filing: 01.05.2013
(51) Int. Cl.: G06Q 50/24, G06Q 50/22

(54) **INFORMATION PROCESSING DEVICE, INFORMATION PROCESSING SYSTEM, INFORMATION PROCESSING METHOD, AND PROGRAM**

(30) Priority: 11.06.2012 JP 2012131995
(71) Applicant: Sony Corporation, Tokyo 108-0075 (JP)
(72) Inventor: KYUSOJIN, Hiroshi, Tokyo 108-0075 (JP); YAMANE, Kenji, Tokyo 108-0075 (JP); TAGAMI, Naoki, Tokyo 108-0075 (JP); WATANABE, Hirofumi, Tokyo (JP)
(74) Representative: Ealey, Douglas Ralph
(86) International application number: PCT/JP2013/002909
(87) International publication number: WO 2013/186979

(57) **Abstract**

[Object] To cause a client termination specialized for image display processing to display a pathological image stored in a server without needing complicated setting processing.

[Solving Means] The information processing apparatus includes a communication unit and a control unit. The communication unit is communicable with an image server apparatus capable of storing a pathological image and another information processing apparatus capable of displaying the pathological image. The control unit is capable of controlling the communication unit to send a display request for sending the pathological image to the other information processing apparatus and causing the other information processing apparatus to display the pathological image, to the image server apparatus.

## Description

### Technical Field

The present technology relates to an information processing apparatus capable of executing information processing for displaying a digital pathological image with another information processing apparatus, an information processing system including such an information processing apparatus, a information processing method in such an information processing apparatus, and a program.

### Background Art

From the past, in a network system that manages medical information in a hospital or the like, a laboratory information system (LIS) server and picture archiving and communication systems (PACS) are used. The LIS server stores and manages examination information including attribute information of patients such as a patient name and age. The PACS server stores and manages image data (pathological image data) captured with a diagnosis target site of a patient being a subject.

For example, in the above-mentioned network system, a case where a user (doctor) operates a client terminal to display a pathological image stored in the PACS server is assumed. First, the user accesses the LIS server using patient information such as a patient name and a patient number and acquires examination information. This examination information describes what identifier (file name) the captured pathological image is stored with. Thus, the user acquires the pathological image from the PACS server using this file name and displays the pathological image on a display.

In the case where the LIS server and the PACS server are operated as independent systems in this manner, in general, data items between the servers are associated with one another and client software is operated in conjunction therewith in the client terminal. For example, Patent Document 1 below describes that an integrated management server unifies and manages an image management server (PACS server) and an examination management server (LIS server) using key information.
Patent Document 1: Japanese Patent Application Laid-open No. 2010-128782

### Summary of Invention

### Problem to be solved by the Invention

However, in the case where the client terminal includes software adapted for both the LIS and PACS, pathological images has a huge data size, and hence cannot be processed at high speed.

In view of the above-mentioned circumstances, it is an object of the present technology to provide an information processing apparatus capable of causing a client termination specialized for image display processing to display a pathological image stored in a server without needing complicated setting processing, an information processing system, an information processing method, and a program.

### Means for solving the Problem

In order to achieve the above-mentioned object, an information processing apparatus according to an embodiment of the present technology includes a communication unit and a control unit. The communication unit is communicable with an image server apparatus capable of storing a pathological image and another information processing apparatus capable of displaying the pathological image. The control unit is capable of controlling the communication unit to send a display request for sending the pathological image to the other information processing apparatus and causing the other information processing apparatus to display the pathological image, to the image server apparatus.

With this configuration, by sending the display request to the image server, the information processing apparatus is capable of causing the other information processing apparatus specialized for image display processing to display the pathological image stored in the image server without needing complicated setting processing.

The communication unit may be communicable with an information server apparatus capable of storing examination information of a patient relating to the pathological image, the examination information including identification information for identifying the pathological image. In this case, the control unit may receive the examination information from the information server apparatus and control the communication unit to send the display request using the identification information included in the received examination information.

With this, the information processing apparatus is capable of causing the other information processing apparatus to display, based on the examination information of the patient, the pathological image corresponding thereto.

The pathological image may be a part of an entire image. In this case, the control unit may add, to the display request, information indicating a position and a size of the displayed pathological image in the entire image.

With this, the information processing apparatus is capable of causing the other information processing apparatus to display the pathological image at arbitrary coordinates and magnification.

The information processing apparatus may further include an output unit that is capable of outputting a user interface of an application including link information corresponding to a position of the pathological image and an operation reception unit that is capable of receiving an operation of a user with respect to the user interface. In this case, the control unit may control, when the operation of the user with respect to the link information is received, the communication unit to send the display request relating to the pathological image corresponding to the link information.

With this, the information processing apparatus is capable of sending the display request also according to an operation executable by presentation software, browser software, or the like in a general personal computer environment, for example, an operation (e.g., click operation) on the link information.

The information processing apparatus may further include an output unit that is capable of outputting a predetermined image relating to the pathological image. In this case, the control unit may control the communication unit to send the display request such that the pathological image is displayed in association with the output predetermined image.

With this, the information processing apparatus is capable of causing the other information processing apparatus to operate as a slave terminal of the information processing apparatus that is a master terminal and the other information processing apparatus is capable of displaying, in association with an image displayed in the information processing apparatus, an associated pathological image. Here, the predetermined image may be a pathological image or may be a menu image for displaying the pathological image.

The control unit may control the output unit to output an entire image including a part of the pathological image as the predetermined image. In this case, the control unit may add, to the display request, information indicating a position and a size of the pathological image in the entire image, the pathological image being displayed by the other information processing apparatus as a part of the entire image.

With this, the information processing apparatus is capable of causing the other information processing apparatus to display the pathological image as the part of the pathological image displayed by the information processing apparatus itself in association therewith.

The control unit may control the output unit to output, as the predetermined image, an image of a first slice of consecutive slices of a predetermined specimen, which is dyed with the first color. In this case, the control unit may control the communication unit to send, as the pathological image, the display request for displaying an image of a second slice of the consecutive slices, which is dyed with a second color different from the first color, to the image server apparatus.

With this, the information processing apparatus is capable of causing the other information processing apparatus to display a different dyeing image of the pathological image displayed by the information processing apparatus itself in association therewith.

The control unit may control the output unit to output, as the predetermined image, a first image of an entire image including the pathological image as a part, the first image including a first coordinate as a center. In this case, the control unit may control the communication unit to send, as the pathological image, the display request for displaying a second image of the entire image, to the image server apparatus, the second image including a second coordinate at a predetermined distance from the first coordinate as a center.

With this, the information processing apparatus is capable of causing the other information processing apparatus to display a pathological image having center coordinates offset by a predetermined distance from the center coordinates of the pathological image displayed by the information processing apparatus itself in association therewith.

The other information processing apparatus may include a plurality of other information processing apparatuses. In this case, the control unit may control the output unit to output, as the predetermined image, the pathological image to which annotation information is added. In this case, the control unit may control the communication unit to send a display switching request for switching on/off display of the annotation information on the pathological image displayed by the other information processing apparatus, to the image server apparatus.

With this, the information processing apparatus is capable of functioning as a teacher apparatus and causing the other information processing apparatus to function as a student apparatus. That is, the information processing apparatus outputs a pathological image with annotation such that the user serving as a teacher advance a lecture. Meanwhile, the information processing apparatus switch on/off the display of the annotation information in the other information processing apparatus for a student, such that the annotation information can be displayed in the student apparatus as a solution of a question at suitable timing.

An information processing system according to another embodiment of the present technology includes a server apparatus, a first information processing apparatus, and a second information processing apparatus.

The server apparatus includes a storage unit that is capable of storing a pathological image, a first communication unit that is communicable with the first information processing apparatus and the second information processing apparatus, and a first control unit that is capable of controlling the first communication unit to send the stored pathological image to the second information processing apparatus in response to a request of the first information processing apparatus.

The first information processing apparatus includes a second communication unit that is communicable with the server apparatus and the second information processing apparatus, and a second control unit that is capable of controlling the second communication unit to send a display request for sending the pathological image to the second information processing apparatus and causing the second information processing apparatus to display the pathological image, to the server apparatus.

The second information processing apparatus includes a third communication unit that is communicable with the server apparatus and the first information processing apparatus, an output unit, and a third control unit that is capable of controlling, in response to the display request, the third communication unit to receive the pathological image sent from the server apparatus and controlling the output unit to output the received pathological image.

An information processing method according to still another embodiment of the present technology includes: receiving identification information for identifying a pathological image stored in a server apparatus; and sending a display request for sending the pathological image to another information processing apparatus and causing the other information processing apparatus to display the pathological image, to the server apparatus.

A program according to a still another embodiment of the present technology causes an information processing apparatus to execute: a step of receiving identification information for identifying a pathological image stored in a server apparatus; and a step of sending a display request for sending the pathological image to another information processing apparatus and causing the other information processing apparatus to display the pathological image, to the server apparatus.

### Effect of the Invention

As described above, according to the present technology, it is possible to cause a client termination specialized for image display processing to display a pathological image stored in a server without needing complicated setting processing.

### Brief Description of Drawings

[Fig. 1] A view showing a configuration of a digital pathological display system according to a first embodiment of the present technology.
[Fig. 2] A block diagram showing a configuration of hardware of an LIS client terminal in the system.
[Fig. 3] A view showing an example of a table showing a correspondence between client terminals that is stored in a PACS server in the first embodiment of the present technology.
[Fig. 4] A view showing an image pyramid structure for explaining a display principle of pathological images handled by the system.
[Fig. 5] A view for explaining a procedure when an image group of the pathological images handled by the system is generated.
[Fig. 6] A flowchart showing a flow of operations of the system according to the first embodiment of the present technology.
[Fig. 7] A view showing a configuration of a digital pathological display system according to a second embodiment of the present technology.
[Fig. 8] A flowchart showing a flow of operations of the system according to the second embodiment of the present technology.
[Fig. 9] A view showing a configuration of a digital pathological display system according to a third embodiment of the present technology.
[Fig. 10] A flowchart showing a flow of operations of the system according to the third embodiment of the present technology.
[Fig. 11] A view showing an example of a screen displayed in each client terminal in the case where a display data type is a "partial display" in the third embodiment of the present technology.
[Fig. 12] A view showing an example of a screen displayed in each client terminal in the case where the display data type is a "different dyeing synchronization display" in the third embodiment of the present technology.
[Fig. 13] A view showing an example of a screen displayed in each client terminal in the case where the display data type is an "offset display" in the third embodiment of the present technology.
[Fig. 14] A view showing an example of a screen displayed in each client terminal in the case where the display data type is the "offset display" in the third embodiment of the present technology.
[Fig. 15] A view showing an example of a table showing a correspondence between client terminals that is stored in the PACS server in order to realize the displays of Figs. 11 to 14 in the third embodiment of the present technology.
[Fig. 16] A flowchart showing a flow of operations of a system in the case where the displays of Figs. 11 to 14 are performed in the third embodiment of the present technology.
[Fig. 17] A view showing an example of a screen displayed in each client terminal in the case where a plurality of data types are combined in the third embodiment of the present technology.
[Fig. 18] A view showing another example of a table showing a correspondence between client terminals that is stored in the PACS server in the case of Fig. 17.
[Fig. 19] A view showing a configuration of a digital pathological display system according to a fourth embodiment of the present technology.
[Fig. 20] A view showing an example of a table showing a correspondence between client terminals that is stored in the PACS server in the fourth embodiment of the present technology.
[Fig. 21] A flowchart showing a flow of operations of a system according to the fourth embodiment of the present technology.

### Mode(s) for Carrying Out the Invention

Hereinafter, embodiments of the present technology will be described with reference to the drawings.

### <First Embodiment>

First, a first embodiment of the present technology will be described.

### [Network Configuration of System]

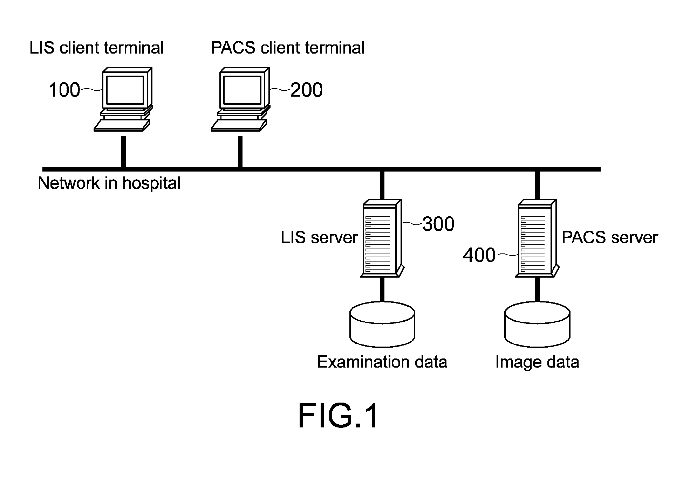

Fig. 1 is a view showing a network configuration of a digital pathological slide display system according to this embodiment.

As shown in the figure, this system is configured as a network in a hospital, for example. The system includes an LIS server 300, a PACS server 400, an LIS client terminal 100, and a PACS client terminal 200. In the figure, the single LIS client terminal 100 and the single PACS client terminal 200 are shown. However, a plurality of LIS client terminals 100 and a plurality of PACS client terminals 200 can be present.

The LIS server 300 stores and manages patient attribution information such as a patient name, a patient number, and age and sex of a patient and examination data such as an examination result and a file name of image data (pathological image data) captured with a diagnosis target site of the patient being a subject during examination.

A PACS server 400 stores and manages the pathological image data with the pathological image data being associated with the patient attribution information.

The LIS client terminal 100 functions as a client terminal of the LIS server 300. Due to software adapted for the LIS, the LIS client terminal 100 is capable of executing processing related to the LIS, for example, receiving the above-mentioned attribution information and examination data from the LIS server 300 and displaying them.

The PACS client terminal 200 functions as a client terminal of the PACS server 400. Due to software adapted for the PACS, the PACS client terminal 200 is capable of receiving the pathological image data from the PACS server 400 and displaying the pathological image data. In particular, in this embodiment, the PACS client terminal 200 includes a highly sophisticated graphics chip or the like and functions as a high-speed viewer dedicated to huge pathological images.

When the PACS client terminal 200 displays a pathological image, the following flow of processes is also conceivable. Specifically, the LIS client terminal 100 receives a file name of the pathological image included in the examination data from the LIS server 300 and notifies the PACS client terminal 200 of it, and the PACS client terminal 200 acquires the pathological image from the PACS server 400 using this file name and displays the pathological image.

However, in such processes, the correspondence relationship between the LIS client terminals 100 and the PACS client terminals 200, that is, which of the LIS client terminals 100 is in charge of display processing (notification processing therefor or the like) in which of the PACS client terminals 200, needs to be set in advance. In particular, in an environment in which a number of LIS client terminals 100 and PACS client terminals 200 are present, such setting processing is very complicated.

In view of this, in this embodiment, as will be described later, information items indicating the correspondence relationship are collectively managed by the PACS server 400 such that complicated setting processing between the client terminals is unified. Further, when receiving the file name of the pathological image from the LIS server 300, the LIS client terminal 100 does not notify the PACS client terminal 200 of it, but uses it to request the PACS server 400 to send the pathological image to the PACS client terminal 200 corresponding to itself and causes the PACS client terminal 200 to display the pathological image. The PACS server 400 sends, based on the information indicating the correspondence relationship, the pathological image to the PACS client terminal 200 corresponding to the file name received upon the request. The request from the LIS client terminal 100 to the PACS server 400 will be referred to as a "display request" in this embodiment.

### [Hardware Configuration of LIS Client Terminal]

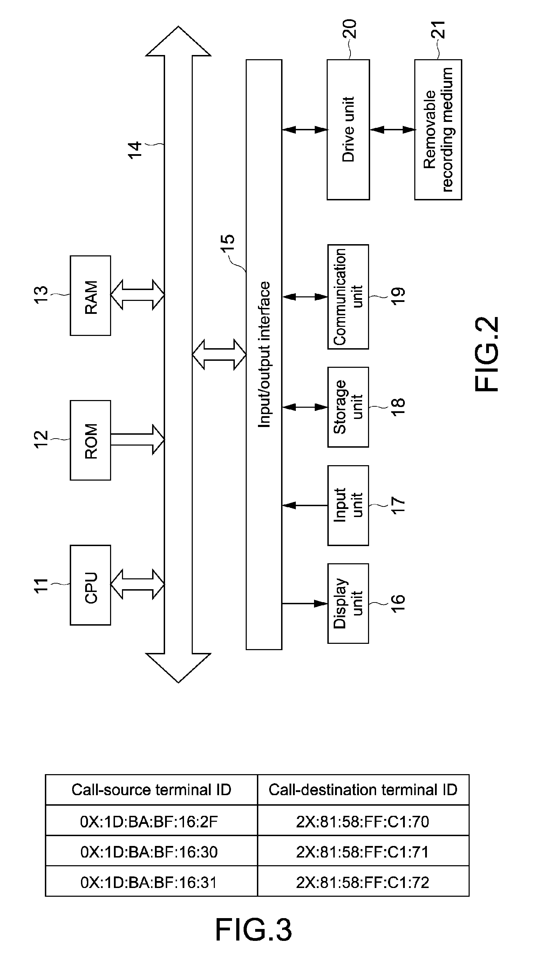

Fig. 2 is a block diagram showing a configuration of hardware of the LIS client terminal 100.

The LIS client terminal 100 includes a central processing unit (CPU) 11, a read only memory (ROM) 12, a random access memory (RAM) 13, an input/output interface 15, and a bus 14 that connects them to one another.

A display unit 16, an input unit 17, a storage unit 18, a communication unit 19, a drive unit 20, and the like are connected to the input/output interface 15.

That is, the LIS client terminal 100 has the same configuration as that of a generally used personal computer (PC).

The display unit 16 is a display device using, for example, a liquid crystal or an electroluminescence (EL).

The input unit 17 is, for example, a pointing device, a keyboard, a touch panel, a microphone, or another operation apparatus. In the case where the input unit 17 includes a touch panel, the touch panel may be integral with the display unit 16.

The storage unit 18 is a non-volatile storage device. The storage unit 18 is, for example, a hard disk drive (HDD), a flash memory, or another solid-state memory. An application program to be executed for displaying the pathological image in the PACS client terminal 200 in this system is also stored in the storage unit 18 in addition to the data of the patient attribution information and the like.

The drive unit 20 is, for example, a device capable of driving a removable recording medium 21 such as an optical recording medium, a floppy (registered trademark) disk, a magnetic recording tape, and a flash memory. In contrast, the storage unit 18 is often used as a device that is installed into the LIS client terminal 100 in advance and mainly drives a non-removable recording medium.

The communication unit 19 is a modem, a router, or another communication apparatus for communicating with a different device, which is connectable to a local area network (LAN), a wide area network (WAN), or the like. The communication unit 19 may perform a wired communication or may perform a wireless communication. The communication unit 19 is often used separately from the LIS client terminal 100.

Although not shown in the figure, hardware configurations of the PACS client terminal 200, the LIS server 300, and the PACS server 400 are basically similar to the hardware configuration of the LIS client terminal 100, and include blocks such as a control unit, a storage unit, and a communication unit that are necessary for them to function as a computer.

It should be noted that, as described above, the PACS client terminal 200 includes a highly sophisticated graphics chip for functioning as a high-speed viewer. For example, the PACS client terminal 200 may be PlayStation (registered trademark) that is a game console manufactured by the present applicant.

Further, the above-mentioned client terminals and servers are shown as stationary apparatuses in Fig. 1. As long as software related to the above-mentioned LIS or PACS can be executed, the client terminals and servers may be, for example, portable apparatuses such as a smart phone, a cellular phone, a tablet PC, and a laptop PC. That is, the above-mentioned client terminal and servers can be any types of information processing apparatuses.

### [Correspondence Relationship between Client Terminals]

Next, a correspondence between the LIS client terminal 100 and the PACS client terminal 200 will be described.

The storage unit of the PACS server 400 stores a table showing the correspondence. Fig. 3 is a view showing an example of the table.

As shown in the figure, this table describes IDs of the LIS client terminals 100 that are call sources of the pathological image and IDs of the PACS client terminals that are call destinations of the pathological image with the IDs being associated with each other.

The ID may be a media access control (MAC) address. A packet of the display request sent by the LIS client terminal 100 includes the MAC address. Therefore, the PACS server 400 is capable of identifying the corresponding PACS client terminal 200 based on the MAC address.

Further, the ID may be a universally unique identifier (UUID). By the LIS client terminal 100 involving its own UUID in the packet of the display request, the PACS server 400 is capable of identifying the corresponding PACS client terminal 200.

### [Structure and Display Principle of Pathological Images]

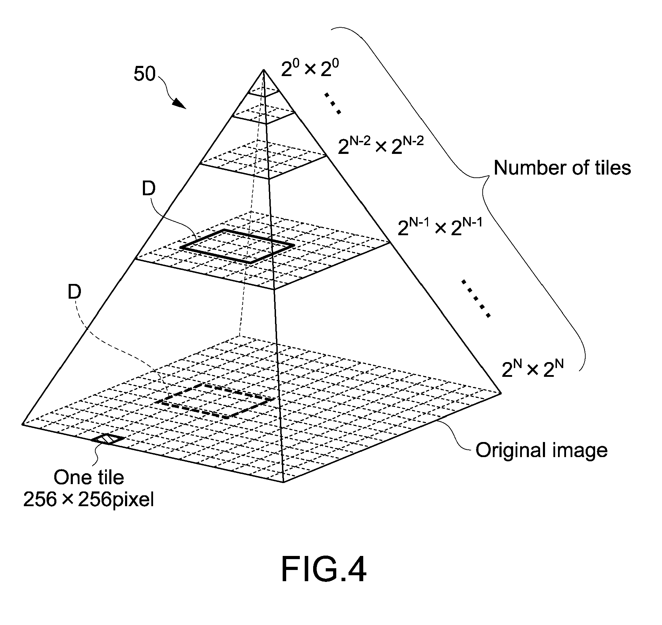

Next, a structure of pathological images stored in the PACS server 400 and displayed in the PACS client terminal 200 and a display principle thereof will be described. Fig. 4 is a view showing an image pyramid structure for explaining the structure and the display principle.

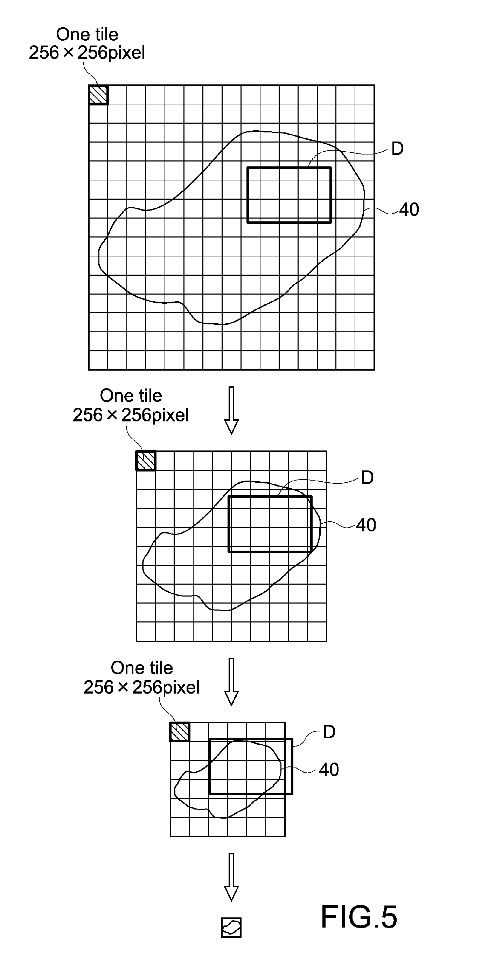

An image pyramid structure 50 in this embodiment is an image group (group of entire images) generated at a plurality of different resolutions with respect to a single pathological image acquired from the same single observation target object 40 (see Fig. 5) by an optical microscope. On a lowermost part of the image pyramid structure 50, a largest image is disposed, and on an uppermost part thereof, a smallest image is disposed. A resolution of the largest image is 50*50 (Kpixel) or 30*40 (Kpixel), for example. A resolution of the smallest image is 256*256 (pixel) or 256*512 (pixel), for example.

That is, when the display unit of the PACS client terminal 200 displays each of those images at, for example, 100% (displays each of those images with same physical dot number as pixel number of image), the largest image is displayed in the largest size and the smallest image is displayed in the smallest size. Here, in Fig. 4, a display area of the display unit is shown by D.

Fig. 5 is a view for explaining a procedure when the image group of the image pyramid structure 50 is generated.

First, a digital image of an original image (huge image) obtained at a predetermined observation magnification by an optical microscope (not shown) is prepared. The original image corresponds to the largest image that is the lowermost image of the image pyramid structure 50 shown in Fig. 4, that is, the image at a highest resolution. Therefore, as the lowermost image of the image pyramid structure 50, an image obtained by the observation at a relatively high magnification by the optical microscope is used.

Note that, in the field of pathology, generally, a matter obtained by slicing an organ, a tissue, or a cell of a living body, or a part thereof is an observation target object 40. Then, a scanner (not shown) having a function of the optical microscope reads the observation target object 40 stored on a glass slide. The obtained digital image is stored in the scanner or another storage apparatus.

As shown in Fig. 5, the scanner generates, from the largest image obtained as described above, a plurality of images whose resolutions are reduced stepwise. Those images are stored in the PACS server 400 in unit of "tile" that is a unit of a predetermined size, for example. The size of one tile is 256*256 (pixel), for example. Each tile is provided with identification information (ID or number) for identifying it.

The thus generated image group forms the image pyramid structure 50, and the image pyramid structure 50 is stored in the storage unit of the PACS server 400. Actually, the PACS server 400 only needs to store the images whose resolutions are different with the images being associated with resolution information items. The process of generating the image pyramid structure 50 may be performed by the PACS client terminal 200.

The entire image group forming the image pyramid structure 50 may be generated by a well-known compression method or may be generated by a well-known compression method used when, for example, a thumbnail image is generated.

Of course, each of the LIS client terminal 100 and the PACS client terminal 200 may download and store at least some of the pathological images stored in the PACS server 400.

The LIS client terminal 100 is capable of causing, according to an operation of the user that is input from the input unit 17, the PACS client terminal 200 to display an arbitrary pathological image extracted from the image pyramid structure 50. At this time, the LIS client terminal 100 is capable of causing the PACS client terminal 200 to display an image of the pathological image at an arbitrary resolution (size) determined based on the file name, which is at an arbitrary site (center coordinates). The LIS client terminal 100 can realize this by adding, to the display request, information on the size and the center coordinates. With this, the user of the PACS client terminal 200 can feel as if he/she actually observes the observation target object 40 while changing the observation magnification. That is, in this case, the PACS client terminal 200 functions as a virtual microscope. Here, the virtual observation magnification actually corresponds to the resolution.

### [Operation of System]

Next, operations of each server and each client in the system configured as described above will be described. In this embodiment and other embodiments, the operations of each server and each client are performed by cooperation of the CPU and software modules executed under the control thereof.

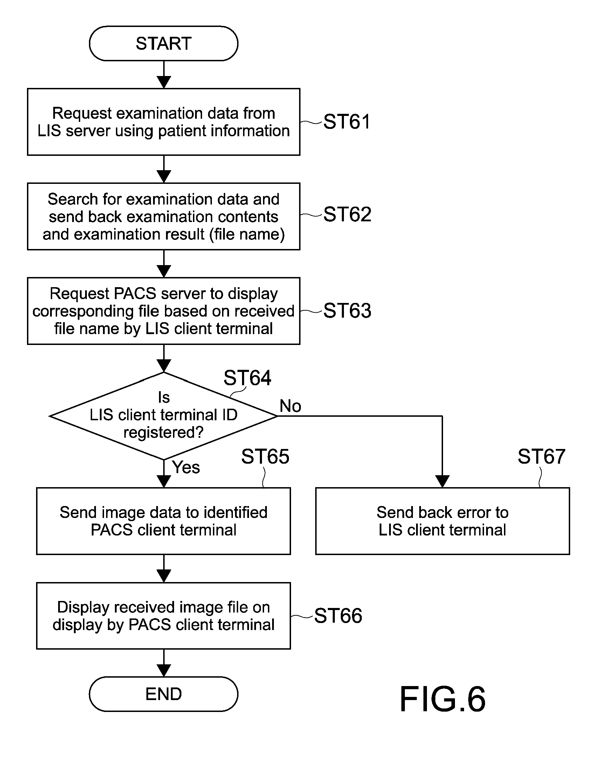

Fig. 6 is a flowchart showing a flow of display processing of the pathological image in the system according to this embodiment. The processes in each of the LIS client terminal 100, the PACS client terminal 200, the LIS server 300, and the PACS server 400 are independently performed. However, for the sake of description, the figure shows these processes in the single flowchart. The same is applied also to flowcharts described in the subsequent figures.

As shown in the figure, the LIS client terminal 100 first requests, using attribution information of a particular patient as a key, examination data of the patient from the LIS server 300 (Step 61).

The LIS server 300 that has received the request of the examination data searches for the examination data of the patient based on the attribution information of the patient, extracts the examination contents and examination result (file name of pathological image) from the storage unit, and sends it back to the LIS client terminal 100 (Step 62).

Subsequently, when receiving the examination data of the file name and the like, the LIS client terminal 100 requests, using this file name as a key, the PACS server 400 to cause the PACS client terminal 200 to display a pathological image file corresponding thereto (Step 63).

The PACS server 400 that has received the display request refers to the table and determines whether or not the ID of the LIS client terminal 100 as the request source is registered (Step 64).

If it is determined that the ID of the LIS client terminal 100 is registered in the table (Yes), the PACS server 400 identifies the PACS client terminal 200 corresponding to the ID in the table and sends pathological image data with the file name to the PACS client terminal 200 (Step 65).

If it is determined that the ID of the LIS client terminal 100 is not registered in the table (No), the PACS server 400 sends back an error to the LIS client terminal 100 as the request source (Step 67) .

Then, the PACS client terminal 200 that has received the pathological image displays the pathological image data on the display unit (Step 66).

### [Effects]

As described above, in this embodiment, the PACS server 400 stores the table showing the correspondence between the LIS client terminal 100 and the PACS client terminal 200. Further, the LIS client terminal 100 sends the display request to the PACS server 400, using the file name acquired from the LIS server 300 as the key. The PACS server 400 sends, based on the table, the pathological image to the PACS client terminal 200 corresponding to the LIS client terminal 100 as the display request source and the PACS client terminal 200 displays this pathological image. With this, the LIS client terminal 100 is capable of causing a desired PACS client to display the pathological image without performing complicated setting processing relating to the correspondence to the PACS client terminal 200.

### <Second Embodiment>

Next, a second embodiment of the present technology will be described. In this embodiment and the following embodiments, the portions not otherwise described have the same configurations as those of the first embodiment and the devices having the same configurations and functions as those of the first embodiment will be denoted by the same reference symbols.

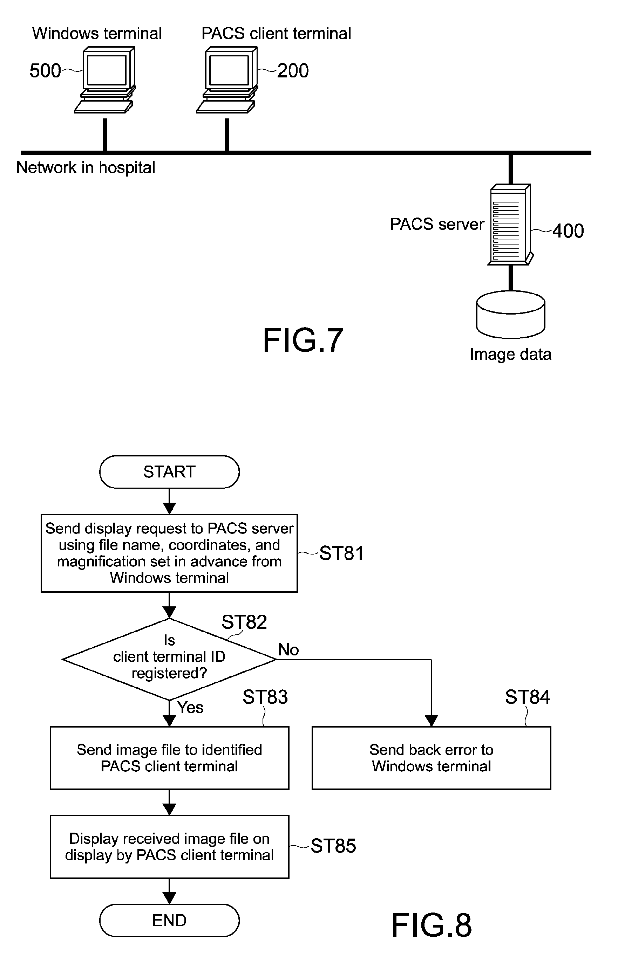

Fig. 7 is a view showing a network configuration of a digital pathological display system according to this embodiment.

In the above-mentioned first embodiment, the display request to the PACS server 400 is sent from the LIS client terminal 100. However, as long as the client terminals can be associated with each other in the PACS server 400, the client terminal that sends the display request does not need to be the LIS client terminal 100.

In view of this, in this embodiment, as shown in Fig. 7, the display request is sent from a generally used Windows (registered trademark) terminal 500 in the hospital network instead of the LIS client terminal 100. Although the LIS client terminal 100 is not shown in this figure and the following figures, it is not shown as the subject that sends the display request and it is actually present in the hospital network. Of course, the OS installed into the terminal 500 is not limited to Windows (registered trademark) and may be different OS such as Mac OS X (registered trademark). That is, the terminal 500 only needs to have a general personal computer environment in which applications such as generally used presentation software and browser software can be executed.

The display request from the Windows terminal 500 may be sent using an application operation made by the user, for example, the user clicking link information in a file generated by presentation software such as Microsoft Power Point (registered trademark) as a trigger.

Fig. 8 is a flowchart showing a flow of operations of the system according to this embodiment.

Preceding the processes in this figure, the user of the Windows terminal 500 first uses the LIS client terminal 100 or the PACS client terminal 200 to refer to a file name of a pathological image displayed in the PACS client terminal 200 and center coordinates and magnification (size) thereof, and sets them in the Windows terminal in advance. Further, the ID of the Windows terminal is also stored in the table while the ID being associated with the ID of the PACS client terminal 200.

Then, the Windows terminal 500 sends a display request to the PACS server 400 using the set file name, coordinates, and magnification (Step 81). The trigger of this sending process may be, as described above, a click operation on a link in a predetermined application file. In this case, data relating to the file name, file position, coordinates, and magnification only needs to be set in this link.

The subsequent processes are the same as the processes in Step 64 and the following steps of Fig. 6 as the first embodiment.

That is, the PACS server 400 that has received the display request refers to the table and determines whether or not the ID of the Windows terminal 500 as the request source is registered (Step 82).

If it is determined that the ID of the Windows terminal 500 is registered in the table (Yes), the PACS server 400 identifies the PACS client terminal 200 corresponding to the ID in the table and sends pathological image data with the file name to the PACS client terminal 200 (Step 83).

If it is determined that the ID of the Windows terminal 500 is not registered in the table (No), the PACS server 400 sends back an error to the Windows terminal 500 as the request source (Step 84).

Then, the PACS client terminal 200 that has received the pathological image displays the pathological image data on the display unit (Step 85).

### <Third Embodiment>

Next, a third embodiment of the present technology will be described.

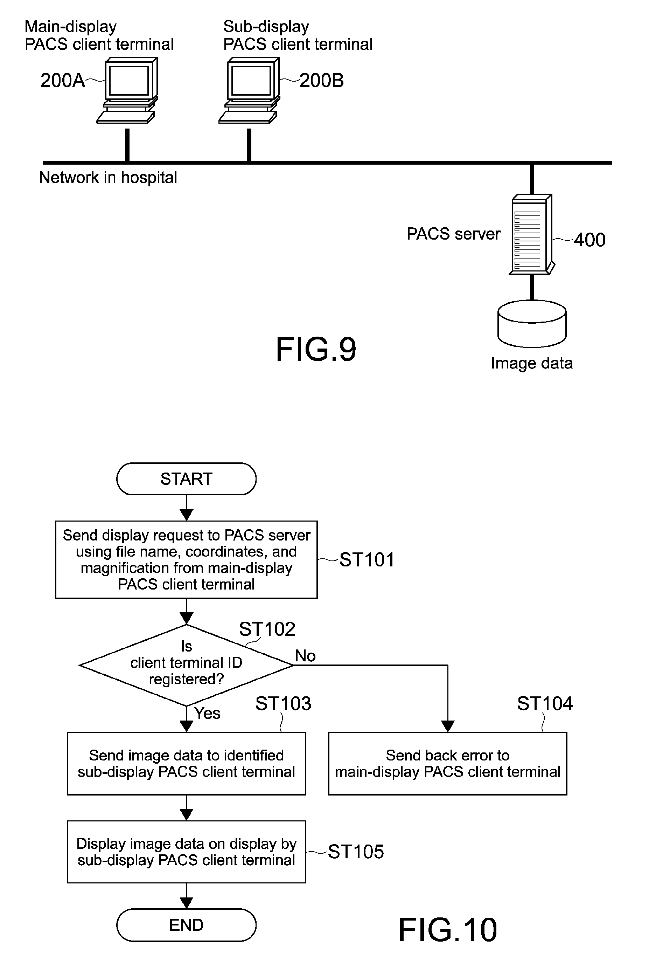

Fig. 9 is a view showing a network configuration of a digital pathological display system according to this embodiment.

Although, in the above-mentioned first and second embodiments, the display request to the PACS server 400 is sent from each of the LIS client terminal 100 and the Windows terminal 500, the display request may be sent from the PACS client terminal 200.

That is, as shown in Fig. 9, for example, one of two PACS client terminals may function as a main-display PACS client terminal 200A and the other may function as a sub-display PACS client terminal 200B.

Such a system can be used, for example, in the case where the user wishes to store, as a snapshot, a certain screen state (pathological image in particular coordinates and at particular magnification) being displayed in the main-display PACS client terminal 200A. In such a case, the main-display PACS client terminal 200A is capable of causing the sub-display PACS client terminal 200B to display the screen and displaying another screen.

In this case, in the table of the PACS server 400, the main-display PACS client terminal 200A and the sub-display PACS client terminal 200B are stored with the main-display PACS client terminal 200A and the sub-display PACS client terminal 200B being associated with each other.

Fig. 10 is a flowchart showing a flow of operations of the system in this case.

As shown in the figure, the main-display PACS client terminal 200A acquires the file name of the pathological image displayed by the main-display PACS client terminal 200A itself and sends a display request including information indicating this file name and the center coordinates and magnification of this pathological image, to the PACS server 400 (Step 101).

The subsequent processes are the same as the processes in Step 64 and the following steps of Fig. 6 as the first embodiment.

That is, the PACS server 400 that has received the display request refers to the table and determines whether or not the ID of the main-display PACS client terminal 200A as the request source is registered (Step 102).

If it is determined that the ID of the main-display PACS client terminal 200A is registered in the table (Yes), the PACS server 400 identifies the sub-display PACS client terminal 200B corresponding to the ID in the table and sends pathological image data with the file name to the sub-display PACS client terminal 200B (Step 103).

If it is determined that the ID of the main-display PACS client terminal 200A is not registered in the table (No), the PACS server 400 sends back an error to the main-display PACS client terminal 200A as the request source (Step 104).

Then, the sub-display PACS client terminal 200B that has received the pathological image displays the pathological image data on the display unit (Step 105).

### (Association Display of Main Client and Sub Client)

In the above-mentioned example, the case where the image displayed in the sub-display PACS client terminal 200B is a still image has been described. However, the image displayed in the sub-display PACS client terminal 200B may be dynamically updated in association with the image displayed in the main-display PACS client terminal 200A.

In this case, as the type of data displayed in the main-display PACS client terminal 200A and the sub-display PACS client terminal 200B, specifically the following three are conceivable.

### (1) Partial Display

An image of an entire pathological slide, a menu screen, and the like are displayed in the main-display PACS client terminal 200A. A portion of them, which the user wishes to display in an enlarged state, is displayed in the sub-display PACS client terminal 200B.

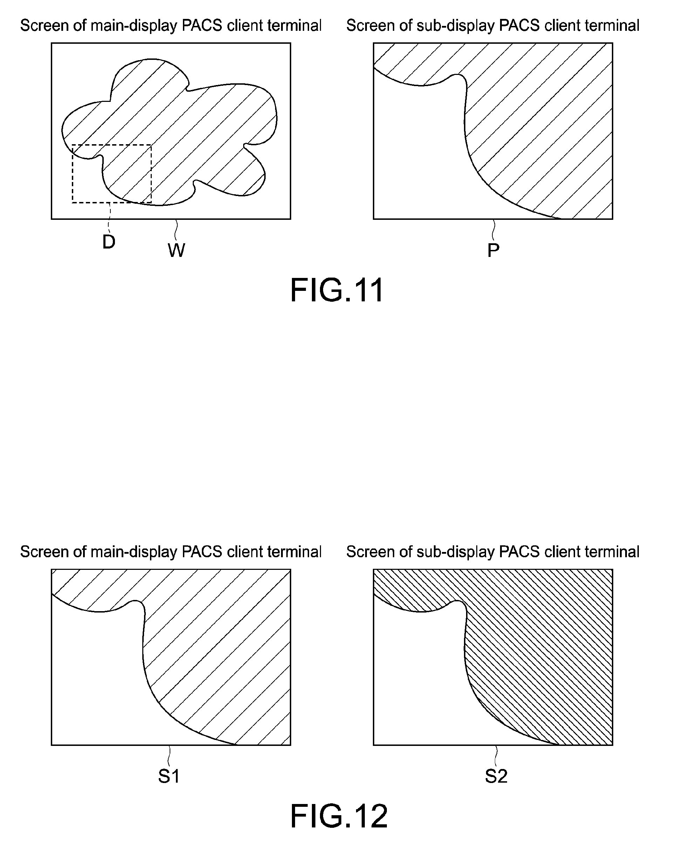

Fig. 11 is a view showing an example of a screen displayed in each of the main-display PACS client terminal 200A and the sub-display PACS client terminal 200B in this case. As shown in the figure, an entire image W of a pathological slide is displayed in the screen of the main-display PACS client terminal 200A. Then, a partial image corresponding to a rectangular region D that is a part thereof is enlarged and displayed as a partial image P in the screen of the sub-display PACS client terminal 200B.

According to an operation of the user, if the rectangular region D is moved or its size is changed in the main-display PACS client terminal 200A, the correspondingly updated image is displayed in the sub-display PACS client terminal 200B.

### (2) Different Dyeing Synchronization Display

If the specimen as the observation target object is one slide of consecutive slices dyed with a certain color and a slide of a slice dyed with a color different from it is also present, one is displayed by the main-display PACS client terminal 200A and the other is displayed by the sub-display PACS client terminal 200B.

Fig. 12 is a view showing an example of a screen displayed in each of the main-display PACS client terminal 200A and the sub-display PACS client terminal 200B in this case. As shown in the figure, an image P1 of the pathological slide dyed with a certain color is displayed in the main-display PACS client terminal 200A. On the other hand, an image P2 of the pathological slide that has the same coordinates and the same magnification as those of the pathological slide image P1 and is dyed with a different color is displayed in the sub-display PACS client terminal 200B.

If the coordinates and size of the pathological slide image S1 are changed in the main-display PACS client terminal 200A, the coordinates and size of the pathological slide image S2 displayed in the sub-display PACS client terminal 200B are also changed in synchronization with it.

### (3) Offset Display

An image having center coordinates offset by a certain value (distance) from a display image of the main-display PACS client terminal 200A is displayed in the sub-display PACS client terminal 200B.

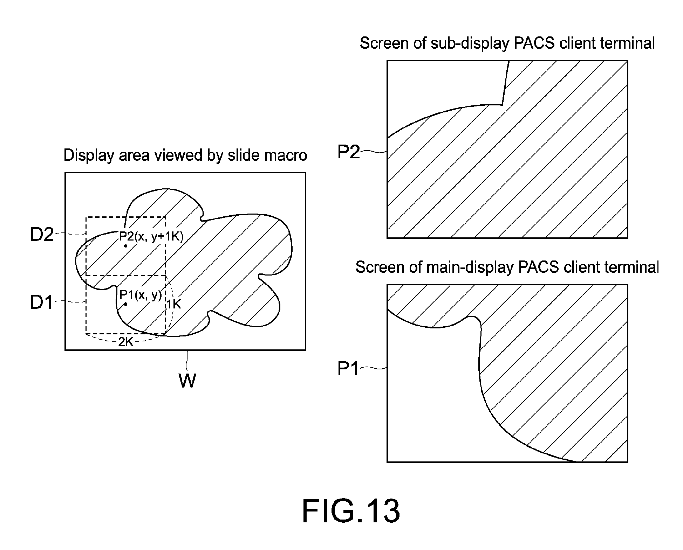

Fig. 13 is a view showing an example of a screen displayed in each of the main-display PACS client terminal 200A and the sub-display PACS client terminal 200B in this case. As shown in the figure, an image P1 of a portion corresponding to a region D1 in the entire image W is displayed in the main-display PACS client terminal 200A. Further, an image P2 of a portion corresponding to a region D2 that is upwardly adjacent to the region D1 in the entire image W is displayed in the sub-display PACS client terminal 200B.

For example, in the case where a display unit of 2K*1K pixels is used, the image P2 obtained by offsetting the image P1 displayed in the main-display PACS client terminal 200A by (0, 1K) in a (X, Y) direction is displayed in the sub-display PACS client terminal 200B.

In addition, a plurality of sub-display PACS client terminals 200B may be used.

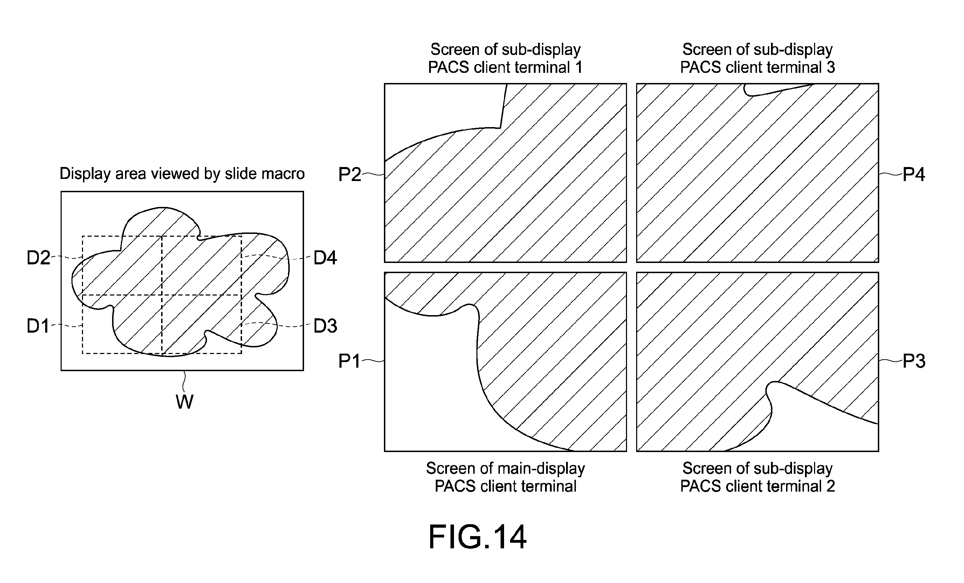

Fig. 14 is a view showing an example of a screen displayed in each of the main-display PACS client terminal 200A and three sub-display PACS client terminals 200B in this case. As shown in the figure, the three sub-display PACS client terminals 200B are used, and hence a 4K*2K display is realized in the case where the single display unit is 2K*1K pixels. That is, images P1 to P4 of portions corresponding to adjacent regions D1 to D4 of 2*2 in the entire image W are displayed in the main-display PACS client terminal 200A and the three sub-display PACS client terminal 200B.

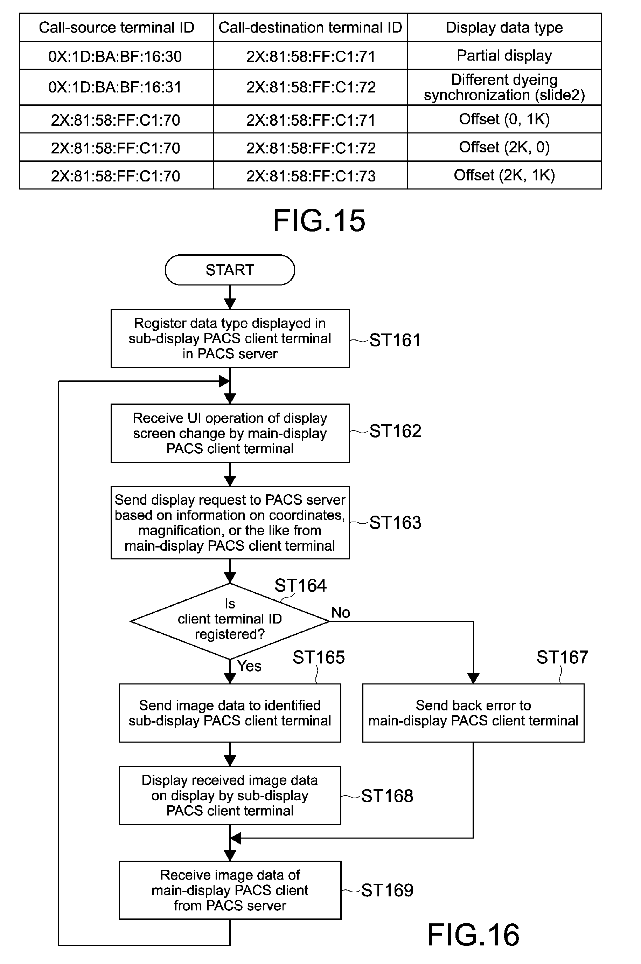

Fig. 15 is a view showing an example of a table showing a correspondence between the client terminals that are stored in the PACS server in order to realize the displays of Figs. 11 to Fig. 14.

As shown in the figure, in addition to the IDs of the clients as shown in Fig. 3 as the first embodiment, any of the three display data types is registered in this table.

In the case where the display data type is the different dyeing synchronization, for example, different numbers are added to the pathological slide images for each color (slide1, slide2, ...). In the table, the number (slide2) of the pathological slide image displayed in the sub-display PACS client terminal 200B is registered together.

In the case where the display data type is the offset display, an offset amount (distance) from the center coordinates of the pathological slide image displayed in the main-display PACS client terminal 200A is registered as a coordinate value together. In the case where the plurality of sub-display PACS client terminals 200B are used for this offset display, the IDs of the plurality of sub-display PACS client terminals 200B are registered in the table while the IDs being associated with the ID of the single main-display PACS client terminal 200A.

Fig. 16 is a flowchart showing a flow of operations of the system in the case where the displays of Figs. 11 to 14 are performed.

As shown in the figure, the main-display PACS client terminal 200A first registers the data type to be displayed by the sub-display PACS client terminal 200B in the table of the PACS server 400 (Step 161). Specifically, the main-display PACS client terminal 200A sends the ID of the sub-display PACS client terminal 200B that displays the pathological slide image in association therewith and information on the display data type of this image to the PACS server 400.

Subsequently, the main-display PACS client terminal 200A receives, through the input unit, from the user, a UI operation of display screen change (Step 162). This UI operation is, for example, a movement of the coordinates of the display position or a change in magnification.

Subsequently, the main-display PACS client terminal 200A sends the display request to the PACS server 400 together with information on the file name acquired in advance and the UI operation (coordinates, magnification, and the like) (Step 163).

The PACS server 400 that has received the display request refers to the table and determines whether or not the ID of the main-display PACS client terminal 200A as the request source is registered (Step 164) .

If it is determined that the ID of the main-display PACS client terminal 200A is registered in the table (Yes), the PACS server 400 identifies the sub-display PACS client terminal 200B corresponding to the ID in the table and sends pathological image data with the file name to the sub-display PACS client terminal 200B (Step 165).

If it is determined that the ID of the main-display PACS client terminal 200A is not registered in the table (No), the PACS server 400 sends back an error to the main-display PACS client terminal 200A as the request source (Step 167).

Then, the sub-display PACS client terminal 200B that has received the pathological image displays the pathological image data on the display unit (Step 168).

Further, if image data for display is necessary also in the main-display PACS client terminal 200A, the image is sent from the PACS server 400 and displayed in the main-display PACS client terminal 200A (Step 169). For example, in the case of the different dyeing synchronization display or in the case of the offset display, the image data is sent also to the main-display PACS client terminal 200A every time the coordinates and size are changed. In the case of the partial display, the entire image has already been downloaded in the main-display PACS client terminal, and hence the image data is not newly sent.

The main-display PACS client terminal 200A and the PACS server 400 follow the UI operation in the main-display PACS client terminal 200A by repeating the above-mentioned processes. Thus, the screen of the sub-display PACS client terminal 200B is updated.

### (Combination of Plurality of Display Data Types)

Alternatively, the plurality of display data types may be combined and displayed in the main-display PACS client terminal 200A and the sub-display PACS client terminal 200B. For example, the partial display and the different dyeing synchronization display may be combined or the partial display and the offset display may be combined.

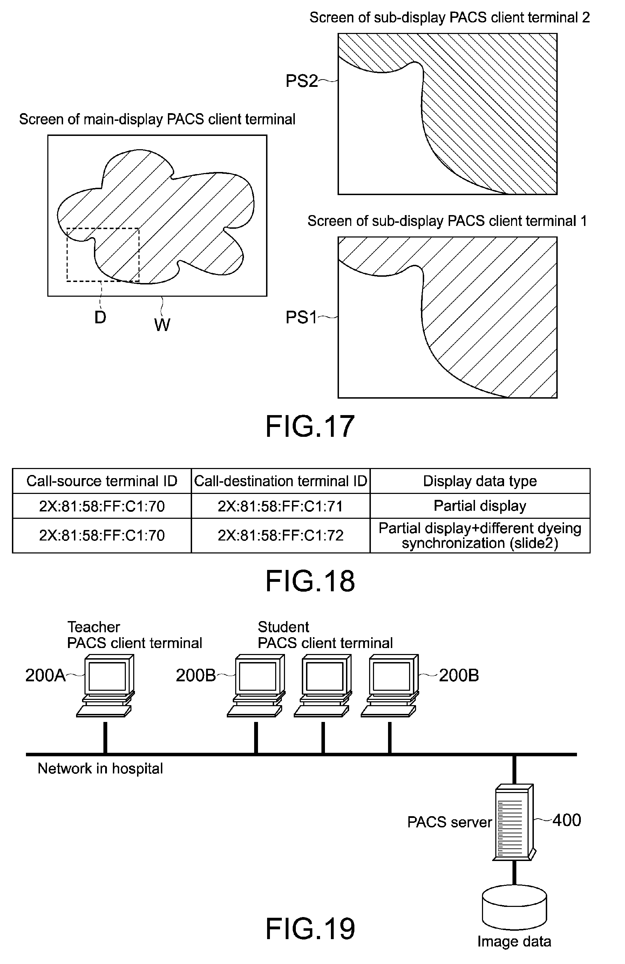

Fig. 17 is a view showing an example of screens displayed in the main-display PACS client terminal 200A and the two sub-display PACS client terminals 200B in the case where the partial display and the different dyeing synchronization display are combined as the display data type.

As shown in the figure, an entire image W dyed with a certain color is displayed in the main-display PACS client terminal 200A. On the other hand, in a first sub-display PACS client terminal 200B, a slide image PS1 of a portion corresponding to a rectangular region D in the entire image W is displayed. In addition, in a second sub-display PACS client terminal 200B, a slide image PS2 dyed with a color different from the color of the entire image W and the partial image PS1, which is a portion corresponding to the rectangular region D in the entire image W.

Fig. 18 is a view showing another example of a table showing a correspondence between the client terminals that is stored in the PACS server 400 in the case where the display combining the display data types as in Fig. 17 is realized.

As shown in the figure, in this table, "partial display" is registered as the display data type of the first sub-display PACS client terminal 200B serving as a first display call destination. On the other hand, "partial display+different dyeing synchronization (slide2)" is registered as the display data type of the second sub-display PACS client terminal 200B serving as a display call destination.

### <Fourth Embodiment>

Next, a fourth embodiment of the present technology will be described.

Fig. 19 is a view showing a network configuration of a digital pathological display system according to this embodiment.

In the above-mentioned third embodiment, the example in which the association display is realized in such a manner that the single PACS client terminal 200 functions as the main terminal and the plurality of PACS client terminals 200 function as the sub terminals due to the one-to-multiple correspondence between the single PACS client terminal 200 and the plurality of PACS client terminals 200 has been shown. However, as shown in the figure, due to this one-to-multiple correspondence, the single PACS client terminal 200A may function as a teacher terminal and other PACS client terminals 200B may function as student terminals in the hospital network.

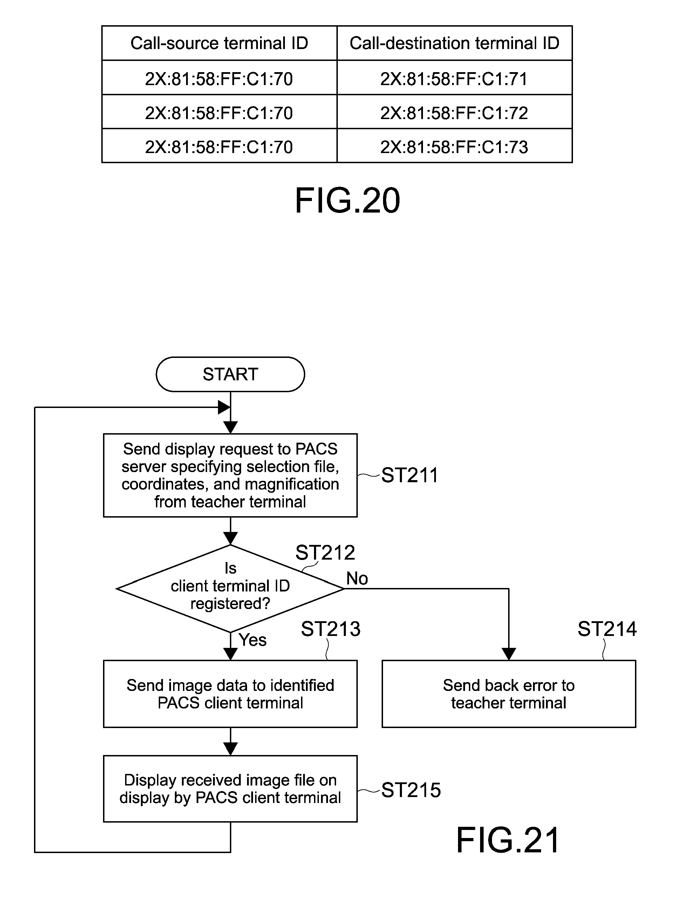

Fig. 20 is a view showing an example of a table showing a correspondence of a single teacher PACS client terminal 200A and three student PACS client terminals 200B. As shown in the figure, the IDs of the three student PACS client terminals 200B are stored while the IDs being associated with the ID of the single teacher PACS client terminal 200A.

Fig. 21 is a flowchart showing a flow of operations of the system in this case.

As shown in the figure, the teacher PACS client terminal 200A acquires the file name of the pathological image displayed by the teacher PACS client terminal 200A itself and sends the display request including the file name and information indicating the center coordinates and magnification of the pathological image, to the PACS server 400 (Step 211).

Subsequently, the PACS server 400 that has received the display request refers to the table and determines whether or not the ID of the teacher PACS client terminal 200A as the request source is registered (Step 212).

If it is determined that the ID of the teacher PACS client terminal 200A is registered in the table (Yes), the PACS server 400 identifies the plurality of student PACS client terminals 200B corresponding to the ID in the table and sends pathological image data with the file name to each of the student PACS client terminals 200B (Step 213).

If it is determined that the ID of the teacher PACS client terminal 200A is not registered in the table (No), the PACS server 400 sends back an error to the teacher PACS client terminal 200A as the request source (Step 214).

Then, each of the student PACS client terminals 200B that has received the pathological image displays the pathological image data on the display unit (Step 215).

This enables the teacher PACS client terminal 200A to control images to be displayed in all the student PACS client terminals 200B.

At this time, the teacher PACS client terminal 200A may constantly display information on a diagnosis, for example, annotation while selectively switching on/off display of the student PACS client terminals 200B.

In this case, the teacher PACS client terminal 200A sends a signal for switching on/off the display together with a display (sending) request of the pathological slide image and annotation information, to the PACS server 400 if necessary. The PACS server 400 correspondingly sends the display switching signal together with the pathological slide image and annotation information to the student PACS client terminals 200B.

With this, a user serving as a teacher can advance a lecture without a memo (annotation) for his/her lecture being seen by users serving as students and display questions and solutions thereof to the student PACS client terminals 200B at suitable timing.

In addition, the on/off of the display of the annotation information is simply controlled according to the display switching signal and the annotation information itself is not sent from the PACS server 400 every time the on/off of the display is performed. Therefore, unnecessary traffic between the PACS server 400 and the student PACS client terminals 200B, which uses the update of the annotation display as a trigger, is reduced.

### [Modified Examples]

The present technology is not limited only to the above-mentioned embodiment and may be variously changed without departing from the gist of the present technology.

In each of the above-mentioned embodiments, the table showing the correspondence between the client terminals is stored in the PACS server 400. However, the storage area of this table is not limited thereto and may be stored in any client or server.

Although, in each of the above-mentioned embodiments, the example in which the present technology is realized over the hospital network, the environment in which the present technology is realized is not limited to the hospital, of course.

In each of the above-mentioned embodiments, the display request to the PACS server is sent using the file name of the pathological image. However, information sent in the display request is not limited to the file name and any type of information may be sent as it is information based on which the pathological image can be identified.

In the above-mentioned third embodiment, as the type of data items displayed by the main-display PACS client terminal and the sub-display PACS client terminal in association with each other, the three examples of the partial display, the different dyeing synchronization display, and the offset display have been shown. However, the association display targets are not limited thereto. For example, examination data (pathological image data) of a certain patient in a certain period may be displayed in the main-display PACS client terminal and examination data of the same patient in a different period may be displayed in the sub-display PACS client terminal.

### [Others]

The present technology may also take the following configurations.
(1) An information processing apparatus, including:
   a communication unit that is communicable with an image server apparatus capable of storing a pathological image and another information processing apparatus capable of displaying the pathological image; and
   a control unit that is capable of controlling the communication unit to send a display request for sending the pathological image to the other information processing apparatus and causing the other information processing apparatus to display the pathological image, to the image server apparatus.
(2) The information processing apparatus according to (1), in which
   the communication unit is communicable with an information server apparatus capable of storing examination information of a patient relating to the pathological image, the examination information including identification information for identifying the pathological image, and
   the control unit receives the examination information from the information server apparatus and controls the communication unit to send the display request using the identification information included in the received examination information.
(3) The information processing apparatus according to (1) or (2), in which
   the pathological image is a part of an entire image, and
   the control unit adds, to the display request, information indicating a position and a size of the displayed pathological image in the entire image.
(4) The information processing apparatus according to any one of (1) to (3), further including
   an output unit that is capable of outputting a user interface of an application including a hyper link indicating position information of the pathological image; and
   an operation reception unit that is capable of receiving an operation of a user with respect to the user interface, in which
   the control unit controls, when the operation of the user with respect to the hyper link is received, the communication unit to send the display request relating to the pathological image corresponding to the hyper link.
(5) The information processing apparatus according to any one of (1) to (4), further including
   an output unit that is capable of outputting a predetermined image relating to the pathological image, in which
   the control unit controls the communication unit to send the display request such that the pathological image is displayed in association with the output predetermined image.
(6) The information processing apparatus according to (5), in which
   the control unit
   controls the output unit to output an entire image including a part of the pathological image as the predetermined image, and
   adds, to the display request, information indicating a position and a size of the pathological image in the entire image, the pathological image being displayed by the other information processing apparatus as a part of the entire image.
(7) The information processing apparatus according to (5) or (6), in which
   the control unit
   controls the output unit to output, as the predetermined image, an image of a first slice of consecutive slices of a predetermined specimen, which is dyed with the first color, and
   controls the communication unit to send, as the pathological image, the display request for displaying an image of a second slice of the consecutive slices, which is dyed with a second color different from the first color, to the image server apparatus.
(8) The information processing apparatus according to any one of (5) to (7), in which
   the control unit
   controls the output unit to output, as the predetermined image, a first image of an entire image including the pathological image as a part, the first image including a first coordinate as a center, and
   controls the communication unit to send, as the pathological image, the display request for displaying a second image of the entire image, to the image server apparatus, the second image including a second coordinate at a predetermined distance from the first coordinate as a center.
(9) The information processing apparatus according to any one of (5) to (8), in which
   the other information processing apparatus includes a plurality of other information processing apparatuses, and
   the control unit
   controls the output unit to output, as the predetermined image, the pathological image to which annotation information is added, and
   controls the communication unit to send a display switching request for switching on/off display of the annotation information on the pathological image displayed by the other information processing apparatus, to the image server apparatus.

### Description of Symbols

- 11: CPU
- 13: RAM
- 16: display unit
- 17: input unit
- 18: storage unit
- 19: communication unit
- 40: observation target object
- 50: image pyramid structure
- 100: LIS client terminal
- 200 (200A, 200B): PACS client terminal
- 300: LIS server
- 400: PACS server
- 500: Windows terminal

## Claims

1. An information processing apparatus, comprising:
a communication unit that is communicable with an image server apparatus capable of storing a pathological image and another information processing apparatus capable of displaying the pathological image; and
a control unit that is capable of controlling the communication unit to send a display request for sending the pathological image to the other information processing apparatus and causing the other information processing apparatus to display the pathological image, to the image server apparatus.

2. The information processing apparatus according to claim 1, wherein
the communication unit is communicable with an information server apparatus capable of storing examination information of a patient relating to the pathological image, the examination information including identification information for identifying the pathological image, and
the control unit receives the examination information from the information server apparatus and controls the communication unit to send the display request using the identification information included in the received examination information.

3. The information processing apparatus according to claim 2, wherein
the pathological image is a part of an entire image, and
the control unit adds, to the display request, information indicating a position and a size of the displayed pathological image in the entire image.

4. The information processing apparatus according to claim 1, further comprising
an output unit that is capable of outputting a user interface of an application including link information corresponding to a position of the pathological image; and
an operation reception unit that is capable of receiving an operation of a user with respect to the user interface, wherein
the control unit controls, when the operation of the user with respect to the link information is received, the communication unit to send the display request relating to the pathological image corresponding to the link information.

5. The information processing apparatus according to claim 1, further comprising
an output unit that is capable of outputting a predetermined image relating to the pathological image, wherein
the control unit controls the communication unit to send the display request such that the pathological image is displayed in association with the output predetermined image.

6. The information processing apparatus according to claim 5, wherein
the control unit
controls the output unit to output an entire image including a part of the pathological image as the predetermined image, and
adds, to the display request, information indicating a position and a size of the pathological image in the entire image, the pathological image being displayed by the other information processing apparatus as a part of the entire image.

7. The information processing apparatus according to claim 5, wherein
the control unit
controls the output unit to output, as the predetermined image, an image of a first slice of consecutive slices of a predetermined specimen, which is dyed with the first color, and
controls the communication unit to send, as the pathological image, the display request for displaying an image of a second slice of the consecutive slices, which is dyed with a second color different from the first color, to the image server apparatus.

8. The information processing apparatus according to claim 5, wherein
the control unit
controls the output unit to output, as the predetermined image, a first image of an entire image including the pathological image as a part, the first image including a first coordinate as a center, and
controls the communication unit to send, as the pathological image, the display request for displaying a second image of the entire image, to the image server apparatus, the second image including a second coordinate at a predetermined distance from the first coordinate as a center.

9. The information processing apparatus according to claim 5, wherein
the other information processing apparatus includes a plurality of other information processing apparatuses, and
the control unit
controls the output unit to output, as the predetermined image, the pathological image to which annotation information is added, and
controls the communication unit to send a display switching request for switching on/off display of the annotation information on the pathological image displayed by the other information processing apparatus, to the image server apparatus.

10. An information processing system, comprising:
a server apparatus;
a first information processing apparatus; and
a second information processing apparatus,
the server apparatus including
a storage unit that is capable of storing a pathological image,
a first communication unit that is communicable with the first information processing apparatus and the second information processing apparatus, and
a first control unit that is capable of controlling the first communication unit to send the stored pathological image to the second information processing apparatus in response to a request of the first information processing apparatus,
the first information processing apparatus including
a second communication unit that is communicable with the server apparatus and the second information processing apparatus, and
a second control unit that is capable of controlling the second communication unit to send a display request for sending the pathological image to the second information processing apparatus and causing the second information processing apparatus to display the pathological image, to the server apparatus,
the second information processing apparatus including
a third communication unit that is communicable with the server apparatus and the first information processing apparatus,
an output unit, and
a third control unit that is capable of controlling, in response to the display request, the third communication unit to receive the pathological image sent from the server apparatus and controlling the output unit to output the received pathological image.

11. An information processing method, comprising:
receiving identification information for identifying a pathological image stored in a server apparatus; and
sending a display request for sending the pathological image to another information processing apparatus and causing the other information processing apparatus to display the pathological image, to the server apparatus.

12. A program that causes an information processing apparatus to execute:
a step of receiving identification information for identifying a pathological image stored in a server apparatus; and
a step of sending a display request for sending the pathological image to another information processing apparatus and causing the other information processing apparatus to display the pathological image, to the server apparatus.
